# EUROPEAN PATENT APPLICATION

(11) **EP 1 437 134 A1**
(43) Date of publication of application: **14.07.2004**
(21) Application number: 02762982.3
(22) Date of filing: 03.09.2002
(51) Int. Cl.: A61K 31/137, A61P 31/16, A61P 43/00

(54) **ANTI-INFLUENZA DRUGS**

(30) Priority: 04.09.2001 JP 2001267236
(71) Applicant: Boehringer Ingelheim International GmbH, 55216 Ingelheim (DE); Techno Network Shikoku Co., Ltd., Takamatsu-shi, Kagawa 760-0033 (JP)
(72) Inventor: KIDO, Hiroshi, c/o The University of Tokushima, Tokushima-Shi, Tokushima 770-0042 (JP)
(74) Representative: Kompter, Hans-Michael, Dr.
(86) International application number: PCT/JP2002/008940
(87) International publication number: WO 2003/020258

(57) **Abstract**

An anti-influenzal agent comprises, as an effective component, ambroxol, bromhexin or a pharmaceutically acceptable salt thereof. This agent is characterized in that it has an anti-influenzal effect through the promotion of the secretion of biological factors, which possess influenza virus-proliferation-inhibitory effect and are included in the fluid secreted in the respiratory tract. It is also characterized in that it can inhibit the influenza virus-proliferation in the respiratory tract through promoting the secretion of substances capable of inhibiting proteases in the respiratory tract, which induce the influenza virus infection, that it can inhibit the influenza virus-proliferation in the respiratory tract through promoting the secretion of mucosal immune substances or IgA and IgG and that it can inhibit any release of inflammatory cytokines in the respiratory tract. The present invention also relates to an agent for treating or preventing influenza virus-infectious diseases.

## Description

### Background of the Invention

The present invention relates to an anti-influenzal agent. More specifically, the present invention pertains to an agent for treating or preventing influenza virus- infectious diseases.

Influenza virus is one of the most commonly encountered infectious pathogen and it may become a cause of considerably high prevalence and mortality rate, in particular, in the aged, the infant, persons suffering from chronic diseases, and persons suffering from immune deficiency diseases (Ref. 1,2).

There have been reported three types of influenza viruses A, B and C. In this respect, it has been known that the A type influenza virus widely prevails and shows strong pathogenicity, while the Band C type influenza viruses rather regionally or locally prevail and show weak pathogenicity. However, it may be recognized that there is a common infection mechanism in the infection of these three types of influenza viruses.
Ref.1: Kim HW, Brandt CD, Arrobio JO, Murphy B, Chanock RM, Parrott RH, Influenza A and B virus infection in infants and young children during t he years 1957-1976, Am. J. Epidemiol., 1979, 109: 464-479.
Ref.2: Barker WH, Mullooly JP, Impact of epidemic type A influenza in a def ined adult population, Am. J. Epidemiol., 1980, 112: 798-813.

The pathogenicity of the influenza virus is determined by the polymorphism of the individual virus genome and the trypsin-type proteases secreted by host cells of the respiratory tract, which allow the virus genome invade into the cytoplasm of the host cells. The trypsin-type protease is secreted as a result of the induction of the membrane fusion activity of influenza virus due to the limitative cleavage of mainly viral envelope glycoproteins [hemagglutinin (HA)] and the corresponding fusion of viral membrane and cytoplasmic membrane (Ref. 3-5).
Ref.3: Klenk HD, Garten W, Host cell proteases controlling virus pathogenicity, Trends Microbiol., 1980, 2: 39-43.
Ref.4: Klenk HD, Rott R, The molecular biology of influenza virus pathogeni city, Adv. Virus Res., 1988, 34: 247-281.
Ref. 5: Homma M, Ohuchi M, Trypsin action on the growth of Sendai virus in tissue culture cells, J. Virol., 1973, 12: 1457-1465.

The cleavage of the viral envelope glycoprotein takes place on the membrane of epithelial cells of the respiratory tract and/or the respiratory cavity (Ref. 6, 6).
Ref.6: Kido H, Yokogoshi Y, Sakai K, Tashiro M, Kishino Y, Fukutomi A, Kutunuma N, Isolation and characterization of a novel trypsin-like protease found in rat bronchiolar epithelial Clara cells, J. Biol. Chem., 1992, 267: 13573-13579.
Ref.7: Tashiro M, Yokogoshi Y, Tobita K, Seto JT, Rott R, Kido H, Tryptase Cl ara, an activating protease for Sendai virus in rat lungs, is involved in pneu mopathogenicity, J. Virol., 1992,66: 7211-7216.

The activity of the protease capable of cleaving the viral envelope glycoprotein is strictly controlled by endogeneous inhibitory compounds for the foregoing protease included in the fluids secreted from the respiratory tract such as the mucosal protease inhibitor (MPI; Ref. 8) in the upper respiratory tract and the pulmonary surfactant (PS; Ref. 9). The surfactant protein A (SP-A) included in the PS of the lung belongs to type C lectin to which sialic acid is added and this protein is directly linked to influenza virus HA to thus inhibit any invasion of viruses in cells (Ref. 10). In addition to these compounds included in the secreting fluids of the respiratory tract, the mucosal immune system serves as a principal immunological defensive system for preventing any invasion of viruses into cells and more specifically, the induction of local secretion of immunoglobulins IgA and IgG is closely related to the protection from the influenza virus infection (Refs. 11-13). These results would suggest that the concentrations of these anti-viral bio-protective substances included in the fluids secreted by the respiratory tract determine the susceptibility of individuals to the influenza virus infection.
Ref.8: Beppu Y, Imamura Y, Tashiro M, Towatari T, Ariga H, Kido H, Human Mucus protease inhibitor in airway fluids is a potential defensive compound against infection with influenza A and Sendai viruses, J. Biochem., 1997, 121: 309-316.
Ref.9: Kido H, Sakai K, Kishino Y, Tashiro M, A pulmonary surfactant is a p otential endogenous inhibitor of proteolytic activation of Sendai virus and inf luenza virus, FEBS Lett., 1993, 322: 115-119.
Ref.10: Benne CA, Kraaijeveld CA, van Strijp JAG, Brouwer E, Harmsen M, Verhoef J, van GoldLMG, van Iwaarden JF, Interactions of surfactant protei n A with influenza A viruses: binding and neutralization, J. Infect. Dis., 1995, 171: 335-341.
Ref.11: Liew FY, Russell SM, Appleyard G, Brand CM, Beale J, Cross-protec tion in mice infected with influenza A virus by the respiratory route is correl ated with local IgA rather than serum antibody or cytotoxic T cell reactivity, Eur. J. Immunol., 1984, 14: 350-356.
Ref.12: Tamura S, Funato H, Hirabayash Y, et al., Functional role of respira tory tract hemagglutinin-specific IgA antibodies in protection against influen za, Vaccine, 1990, 8: 479-485.
Ref.13: Wright PF, Murphy BR, Kervina M, Lawrence EM, Phelan MA, Karz on DT, Secretory immunological response after intranasal inactivated influe nza A virus vaccinations: evidence for immunoglobulin A memory, Infect. Im mun., 1983,40: 1092-1095.

Ambroxol (2-amino-3,5-dibromo-N-[trans-4-hydroxy cyclohexyl] benzyl amine) known as an expectorant or a sputum-dissolving agent has been used for treating chronic bronchitis and respiratory distress syndrome of newborn (Ref. 14).

It has been reported that ambroxol has such pharmacological functions as the control of mucus on the adenocyte of the respiratory tract and the promotion of the PS-production (Ref. 15).

Moreover, ambroxol also shows an antioxidant function (Ref. 16) and an anti-inflammatory function associated with the reduction of inflammatory cytokines released from the bronchial alveolar macrophages, monocytes and granulocytes (Refs. 17, 18). However, there has not yet been known any function of ambroxol on the in vivo influenza virus infection.

Bromhexin (2-amino-3,5-dibromo-N-cyclohexyl-N-methylbenzyl amine) known as an expectorant has been used for treating chronic bronchitis. Howe ver, there has not yet been known any function of bromhexin on the in vivo i nfluenza virus infection.
Ref. 14: Germouty J, Jirou-Najou J, Clinical efficacy of ambroxol in the treat ment of bronchial stasis, Respiration, 1987, 51: 37-41.
Ref.15: Heath MF, Jacobson W, The inhibition of lysosomal phospholipase A from rabbit lung by ambroxol and its consequences for pulmonary surfactant, Lung, 1985, 163: 337-44.
Ref.16: Gillissen A, Scharling B, Jaworska M, Bertling A, Rasche K, Schultze -Werninghaus G, Oxidant scavenger function of ambroxol in vitro: a comparis on with N-acetylcysteine AC, Res. Exp. Med. (Berl), 1997, 196: 389-398.
Ref.17: Pfeifer S, Zissel G, Kienast K, Muller-Quernheim J, Reduction of cyto kine release from blood and bronchoalveolar mononuclear cells by ambroxol, Eur. J. Med. Res., 1997, 2:129-132.
Ref.18: Gibbs BF, Schmutzler W, Vollrath IB, Brostharardt P, Braam U, Wolf f HH, Zadlo-Klarwasser G, Ambroxol inhibits the release of histamine, leuko trienes and cytokines from human leukocytes and mast cells, Inflamm. Res., 1999, 48: 86-93.

### Disclosure of the Invention

It is an object of the present invention to provide an anti-influenzal agent or an agent for treating or preventing influenza virus infectious diseases, while making use of the defensive effect, against the influenza virus infection, of ambroxol and/or bromhexin, which has an antioxidant effect and can serve as a sputum-dissolving agent capable of promoting the release of PS.

The gist of the present invention resides in an anti-influenzal agent comprising, as an effective component, ambroxol, or a pharmaceutically acceptable salt thereof.

The agent of the present invention is characterized in that it possesses an anti-influenzal effect through promoting the secretion of a biological or in vivo factors (a group of bio-protective substances) showing an anti-influenza virus function and included in the fluid secreted from the respiratory tract. In other words, the present invention herein provides an anti-influenzal agent characterized in that the agent comprises, as an effective component, ambroxol, bromhexin or a pharmaceutically acceptable salt thereof and that it possesses an anti-influenzal effect through promoting the secretion of a biological or in vivo factors (a group of bio-protective substances) showing an anti-influenza virus function and included in the fluid secreted from the respiratory tract.

Moreover, the agent of the present invention is characterized in that the proliferation of influenza virus in the respiratory tract is controlled by promoting the secretion of substances capable of inhibiting the protease present in the respiratory tract, which can induce the infectiousness of influenza virus and the mucosal immune substances such as IgA and IgG and more specifically, the present invention herein provides an anti-influenzal agent characterized in that the anti-influenzal agent comprises, as an effective component, ambroxol, bromhexin or a pharmaceutically acceptable salt thereof and that the proliferation of influenza virus in the respiratory tract is controlled by promoting the secretion of biological factors showing anti-influenza virus function and included in the fluid secreted from the respiratory tract, such as MPI and PS, and the mucosal immune substances such as IgA and IgG.

Further, the agent of the present invention is characterized in that it can inhibit the release of inflammatory cytokines in the respiratory tract and more specifically, the present invention also provides an anti-influenzal agent characterized in that the agent comprises, as an effective component, ambroxol, bromhexin or a pharmaceutically acceptable salt thereof and that it can inhibit the proliferation of influenza virus through promoting the secretion of anti-influenza virus factors included in the fluids secreted in the respiratory tract such as MPI and/or PS as well as the secretion of mucosal immune substances such as IgA and/or IgG and that it can inhibit the release of inflammatory cytokines in the respiratory tract.

The anti-influenzal agent of the present invention serves as an agent for treating or preventing influenza virus-infectious diseases.

The present invention also provides use of ambroxol, bromhexin or a pharmaceutically acceptable salt thereof for the preparation of anti-influenzal agent.

The present invention further provides a method of treating influenza virus-infectious diseases which comprises administering an anti-influenzal agent comprising ambroxol, bromhexin or a pharmaceutically acceptable salt thereof as an effective component to patients suffering from the diseases.

### Brief Description of the Drawings

Fig. 1 is a diagram showing the fact that ambroxol improves the surviving rate of mice infected with influenza A virus.
Fig. 2 is a diagram for illustrating the effect of ambroxol to inhibit the viral proliferation in BALF (A) or the pulmonary lesions visually observed in mice after 4 days from the infection with the virus (B).
Fig. 3 is a diagram for illustrating the effect of ambroxol to stimulate the secretion of the mucous immunoglobulin IgA in BALF of a group of un-infected mice (A), or a group of mice infected with influenza A virus (B).
Fig. 4 is a diagram for illustrating the effect of ambroxol to stimulate the secretion of the mucous immunoglobulin IgG in BALF of a group of un-infected mice (A), or a group of mice infected with influenza A virus (B).

### Best Mode for Carrying Out the Invention

Regarding ambroxol (2-amino-3,5-dibromo-N-[trans-4-hydroxy cyclohexyl] benzyl amine), the hydrochloride thereof represented by the following chemical formula 1 (general name: ambroxol hydrochloride; chemical name: trans-4-[(2-amino-3,5- dibromo-benzyl) amino] cyclohexanol hydrochloride) has widely been used in the world including Germany as an expectorant or a sputum-dissolving agent and has been used for treating chronic bronchitis and respiratory distress syndrome of newborn, as has been discussed above.

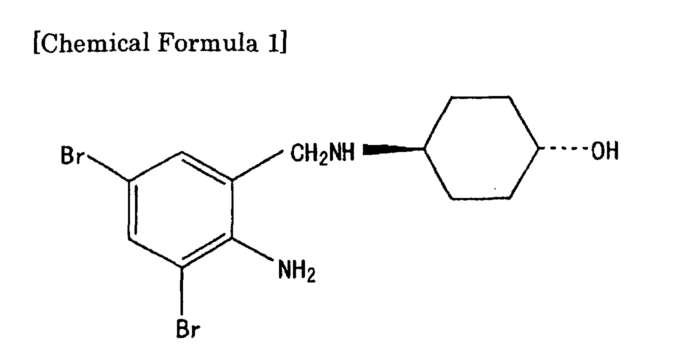

Also, regarding bromhexin (2-amino-3,5-dibromo-N-cyclohexyl-N-methylbenzyl amine), the hydrochloride thereof represented by the following chemical formula 2 (general name: bromhexin hydrochloride; chemical name: 2-amino-3,5-dibromo-N-cyclohexyl-N-methylbenzyl amine hydrochloride) has widely been used in the world including Germany as an expectorant and has been used for treating chronic bronchitis, as has been discussed above.

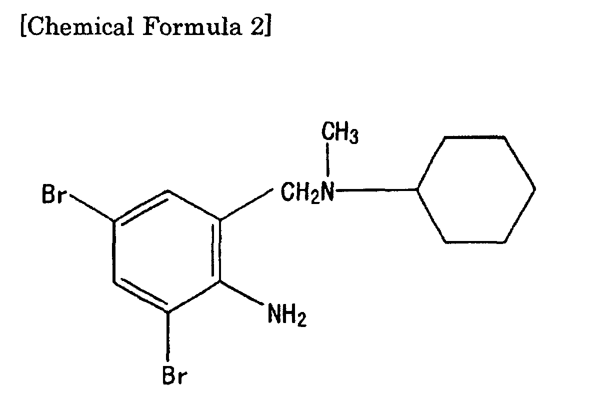

The viral infectious diseases capable of being treated with and/or prevented by the agent according to the present invention may be any one inasmuch as they are caused through the infection of the respiratory tract with viruses having outer membrane glycoproteins and specific examples thereof are diseases attributable to influenza viruses, para-influenza viruses, respiratory syncytial viruses, measles viruses or mumps viruses.

The anti-influenzal agent of the present invention can be administered to a patient in a variety of dosage forms, like the usual pharmaceutical compositions, for instance, orally administered solid pharmaceutical preparations such as tablets, powders, fine granules, granules, capsules, suspensions, troches and chewable preparations, and liquid preparations such as elixirs and syrups (including dry syrups). Alternatively, if the oral administration thereof is ill-fitted for a patient or in case where it is desired to ensure more rapid and reliable efficacy through the selection of local administration, the anti-influenzal agent of the present invention is administered according to the methods conventionally used such as injection of liquid preparations, spraying of mist, injection using a nebulizer, the administration by a dry powder device (DPD) using a spinhaler or a diskhaler or the administration by a metered dose inhaler (MDI). In this respect, these methods are selected and used while taking into consideration, for instance, facilities, reliability and effectiveness.

The dose or dosage, to be administered, of the anti-influenzal agent of the invention may appropriately be controlled depending on the dosage forms of the desired pharmaceutical preparations.

The anti-influenzal agent of the invention may be administered to a patient in a daily dose in portions over one or several times per day if it is in the dosage form of an orally administered solid preparation such as a tablet or an orally administered liquid preparation. In case of the dosage forms, for infants, to be taken at one dose, such as a syrup, a troche and a chewable tablet, which are pharmaceutical preparations for simultaneously enjoying their local effects and systemic effects through the internal use thereof, it is sufficient to incorporate 1/2 to 1/10 time the daily dose into the agent in the foregoing dosage forms prior to use the same. In this case, the total dose thereof may be less than the daily dose. Contrary to this, such an amount of the effective component as that corresponding to the daily dose may be formulated into a single dose, inasmuch as it is not unreasonable from the viewpoint of the dosage form of the pharmaceutical preparation. In addition, in case of, for instance, the administration of an injectable liquid preparation, an agent administered by a mist-spray device, the administration by a nebulizer or the administration by the powder inhalation, the agents may be prepared in such a manner that they contain the effective component in an amount of 1/10 to 1/100 time the dose for the orally administered agent for internal use.

In the preparation of these agents, a variety of currently used additives may be employed, such as a filler, a thickening agent, a binder, a disintegrator, a surfactant, a lubricant, a coating agent, a sustained release agent, a diluent and/or an excipients. In addition to the foregoing, the agent of the present invention may, if necessary, further comprise other additives such as a solubilizing agent, a buffering agent, a preservative, a solubilizer, an isotonicity, an emulsifying agent, a suspending agent, a dispersant, a thickener, a gelatinizing agent, a hardening agent, an absorbent, an adhesive, an elasticizing agent, an adsorbent, a perfume, a coloring agent, a corrigent, an antioxidant, a humectant, a light-screening agent, a brightener and/or an anti-static agent.

More specifically, examples of such additives include an excipient such as lactose, corn starch, mannitol, D-sorbitol, crystalline cellulose, erythritol and sucrose; a binder such as hydroxypropyl cellulose (HPC-L), hydroxypropyl methyl cellulose, polyvinyl pyrrolidone, methyl cellulose and gelatinized starch; a disintegrator such as calcium carboxymethyl cellulose, sodium cross carboxymethyl cellulose and crosslinked polyvinyl pyrrolidone; a lubricant such as magnesium stearate and talc; a perfume, for instance, a flavor or an aromatic oil such as 1-menthol, vanillin, lemon oil, cinnamon oil and mentha oil; and/or an adsorbent such as synthetic aluminum silicate and light anhydrous silicic acid. Moreover, it is also possible to prepare coated pharmaceutical preparations through the use of a currently used coating agent such as hydroxypropyl methyl cellulose, hydroxypropyl cellulose, methyl cellulose or polyvinyl pyrrolidone. If necessary, a sweetener may likewise be used, in particular, in troches, syrups and chewable preparations among others. Specific examples of such sweeteners are mannitol, glucose, maltose, starch syrup, malt extract, maltitol, sorbitol, sucrose, unrefined sugar, fructose, lactose, honey, xylitol, hydrangea tea, saccharin, aspartyl phenylalanine ester and other malto-oligo saccharides, and oligo saccharides such as maltosyl sucrose, isomaltyrose of reduced type and raffinose. Pharmaceutical preparations containing these additives may be prepared according to any method known in this field, currently used ones or ordinary ones depending on the dosage forms thereof.

Regarding the powdery and granular preparations such as the powders, fine granules and granules [including those administered by a metered dose inhaler (MDI) or a dry powder device (DPD)], they may appropriately be prepared, while taking into consideration various properties such as the dustability and adhesiveness. For instance, they are preferably prepared, while taking into consideration physical properties thereof such as the bulk, dustability, adhesiveness, hygroscopicity, charging ability, wettability and solubility of each powdery substance as well as other properties such as the particle size (particle diameter), surface area and shapes of particles. Specifically, in the powder inhalation, one should pay a special attention to the particle size of the drug components in order to effectively make the drug arrive at the affected site and accordingly, the most suitable particle size thereof should range from 0.5 to 5.0 µm. Moreover, it is also preferred to prepare the agent while taking into consideration, for instance, the easy handling ability, and prevention of hygroscopicity, decomposition behaviors, denaturation and discoloration. The powder may be prepared according to any known pulverization method such as dry pulverization, wet pulverization, low temperature pulverization, jet pulverization, batchwise pulverization, continuous open circuit- pulverization and continuous closed circuit-pulverization methods, which may be used alone or in any combination, depending on purposes.

It is recognized that ambroxol and bromhexin shows an effect of promoting the secretion of antiviral factors in the respiratory tract and in turn has an effect of inhibiting the proliferation of influenza viruses in the respiratory tract. The effect of ambroxol would be proved by referring to the facts that it can increase the concentrations of virus proliferation-inhibitory substances such as SP-A, MPI, IgA and IgG in the respiratory tract and that it can inhibit the release of inflammatory cytokines in the fluid secreted in the respiratory tract.

### Examples

The present invention will hereunder be described in more detail with reference to the following Examples, but the present invention is not restricted to these specific Examples at all.

### Example

### <Summary>

Ambroxol serving as a sputum-dissolving agent, which had an antioxidant action and induced the release of PS was inspected for the effect of protecting a subject from the infection with influenza virus, using mice.

After infecting the nasal cavities of mice with a lethal dose of influenza A/Aichi68 (H3N2) viruses, ambroxol or an excipient was intraperitoneally administered to those mice twice a day and then there were determined or analyzed the surviving rate, the titer of virus in BALF, the cytokines and antiviral factors present in BALF or the mucosal immunoglobulins IgA and IgG, as well as the concentrations of PS and MPI.

As a result, it was found that ambroxol significantly inhibited the proliferation of the virus and considerably improved the surviving rate of the infected mice. Regarding the determination of the surviving rate, the effect of ambroxol reached the maximum level at a dose of 10 mg/kg/day and was reduced at the dose higher than the dose specified herein. In case of such a higher dose, however, the surviving rate of mice was improved as compared with that observed for the physiological saline-administered animal group used as a control. The infection with influenza virus induced the release of antiviral factors and inflammatory cytokines in the fluid secreted from the respiratory tract and ambroxol further promoted the release of these antiviral factors. However, it also promoted the release of a trypsin-type protease, which would promote the virus-proliferation. Moreover, ambroxol temporarily inhibited the release of cytokines or tumor necrosis factor- α (TNF- α), interferon- γ (IFN- γ) and interleukin-2 (IL-2) in the fluid secreted in the respiratory tract.

It has been recognized that ambroxol has several negative effects involved in the virus proliferation in vivo, but as a whole, it can significantly increase the level of a group of substances in the respiratory tract, which can control the virus proliferation and this clearly suggests that it can clinically be applied as an effective agent for treating a patient infected with influenza virus.

After infecting the nasal cavities of mice with a lethal dose of mouse-adaptive type influenza A/Aichi68 (H3N2) viruses, ambroxol was administered to those mice and then there were determined or analyzed the surviving rate, the titer of virus, and the virus proliferation and the concentrations of a trypsin type protease, MPI, PS, IgA, IgG and cytokines in the fluid secreted in the respiratory tract.

### <Subject and Methods>

### 1) Animals and Subjects

Three-week-old female oddY mice each having a body weights ranging from 8 to 10 g and free of any specific pathogen were purchased from Japan SLC Inc. (Shizuoka, Japan). All of the mice were treated on the basis of the Guideline for Animal Experiments, Tokushima University. Boehringer Ingelheim furnished the inventors of this invention with ambroxol. Trypsin originated from the porcine spleen was purchased from Sigma Company. The mouse-adaptive type influenza A/Aichi68 (H3N2) viruses (Ref. 19) were used after the proliferation thereof in 10-day-old embryo-containing eggs.
Ref. 19: Ovcharenko AV, Zhirnov OP, Aprotinin aerosol treatment of influenza and paramyxovirus bronchopneumonia of mice, Antiviral. Res., 1994, 23: 107-118.

### 2) Infection with Virus and Method for Administering Ambroxol

The nasal cavities of mice were infected with the influenza A/Aichi/68 (H3N2) viruses of 6.6 × 10⁴ plaque-forming units (PFU) at a dose of 20 µl, under the anesthetization with ether. Within 10 to 15 minutes immediately after the virus infection, 200 µl of ambroxol mixed with a common salt aqueous solution was intraperitoneally administered to each group (10 animals each) of animals at a dose of 2, 5, 10 or 15 mg/kg body weight. Thereafter ambroxol was administered to these groups of animals according to the same procedures used above twice a day over 7 to 10 days. In experiments for determining or analyzing a variety of compounds present in the fluid secreted from the respiratory tract and a variety of desired pathological changes of the same, three groups of animals were selected to treat them with ambroxol at doses of 0, 10 and 30 mg/kg/day, respectively. Each group comprised 80 animals. The level of viruses present in BALF was determined according to the immunofluorescent cell-counting method) as has previously been reported (Ref. 20).
Ref.20: Tashiro M, Homma M, Pneumotropism of Sendai virus in relation to protease-mediated activation in mouse lungs, Infect. Immun., 1983, 39: 879-888.

### 3) Preparation of BALF

The animals used in this test were divided into the following groups (each including 80 animals): a group treated with ambroxol, a group free of any such treatment, a group infected with influenza virus and a group free of any influenza virus- infection. At least 5 animals were selected from each test group every day to thus collect BALF (according to the method of Singh et al. (Ref. 21)) over 7 days. The samples of BALF were stored at -80°C till they were practically used.
Ref. 21: Singh G, Katyal SL, An immunologic study of the secretory products of rat clara cells, J. Histochem. Cytochem., 1984, 32: 49-54.

### 4) Determination of the level of SP-A, Cytokine and Immunoglobulin in BALF

There is a homology of 95% between the SP-A amino acid sequences of mouse and rat (Refs. 22, 23) and the isolated polyclonal antibody for the rat's SP-A (Refs. 6, 24) reacted with the mouse SP-A. Thus, an enzyme-linked immunosorbent assay (ELISA) system was constructed using specific antibodies for non-biotinylated and biotinylated rat's SP-A and the concentration of the SP-A in mouse BALF was analyzed using mouse SP-A as the reference material for preparing a calibration curve. The levels of cytokines [TNF- α, IL-2, IFN- γ , interleukin-6 (IL-6), interleukin-4 (IL-4)] present in BALF were determined using ELISA kits (available from Bio-Source International, CA, USA) according to the manufacturer's protocol. The levels of IgA and IgG present in BALF were likewise determined using ELISA kits (available from Bethyl Company, TX, USA). In this connection, absorbances observed at 490 nm and 450 nm were read by Immuno Mini NJ-2300 Multi-plate-Reader.
Ref. 21: Singh G, Katyal SL, An immunologic study of the secretory products of rat clara cells, J. Histochem. Cytochem., 1984, 32: 49-54.
Ref.22: Korfhagen TR, Bruno MD, Glasser SW, et al. Murine pulmonary surf actant SP-A: gene cloning, sequence, and transcripitional activity, Am. J. Ph ysiol., 1992, 263: L546-554.
Ref. 23: Lacaze-Masmonteil T, Fraslon C, Bourban J, Raymondjean M, Kahn A, Characterization of the rat pulmonary surfactant protein A promoter, Eur. J. Biochem., 1992, 206: 613-623.
Ref.24: Sakai K, Kweon MN, Kohri T, Kishino Y, Effects of a pulmonary surf actant and surfactant protein A on phagocytosis of fractionated alveolar mac rophages: relationship to starvation, Cell. Mol. Biol., 1992, 38: 123-130.

### 5) Determination of Enzymes and Inhibitors

As has been described above (Kido H, Yokogoshi Y, Sakai K, Tashiro M, Kishino Y, Fukutomi A, Kutunuma N, Isolation and characterization of a novel trypsin-like protease found in rat bronchiolar epithelial Clara cells, J. Biol. Chem., 1992, 267:13573-13579), the trypsin type protease was determined using a sequence: N-tert-butoxycarbonyl-Gln-Ala-Arg-4-metyhl-coumaryl-7-amide similar to the consensus cleavage motif of the influenza virus HA. The inhibitory activity of MPI, which corresponded 90% of the substances inhibiting the influenza virus HA-cleaving proteases present in BALF (Refs. 25, 26) was determined as follows: MPI was treated with a 5% (v/v) perchloric acid solution, while making use of the stability of MPI to acids and heat, the mixture was centrifuged to remove most of the proteins present therein and to collect the supernatant of BALF and then the latter was boiled at 100°C for 10 minutes. Thereafter, the resulting supernatant was centrifuged at 1500 × g for 15 minutes, followed by adjusting the pH thereof to 7.0 with 4M KOH and determination of the protease-inhibitory activity of the supernatant according to the method previously proposed (Beppu Y, Imamura Y, Tashiro M, Towatari T, Ariga H, Kido H, Human Mucus protease inhibitor in airway fluids is a potential defensive compound against infection with influenza A and Sendai viruses, J. Biochem., 1997, 121: 309-316).
Ref.25: Stolk J, Rossie W, Dijkman JH, Apocynin improves the efficacy of a s ecretory leukocyte protease inhibitor in experimental emphysema, Am. J. Re spir. Crit. Care Med., 1994, 150: 1628-1631.
Ref. 26: Ohlsson K, Tegner H, Akesson U, Isolation and partial characterizat ion of a low molecular weight acid stable protease inhibitor from human bron chial secretions, Hoppe Seylers Z. Physiol. Chem., 1977, 358: 583-589.

### <Statistical Treatment>

All of the results obtained are expressed in terms of the average ±SD. The significant difference between the group treated with ambroxol and the group free of such a treatment or the control was evaluated using Paired Student's t-Test and the value P<0.05 was deemed to be significant.

### <Results>

### 1) Ambroxol substantially improved the surviving rate of mice infected with influenza virus.

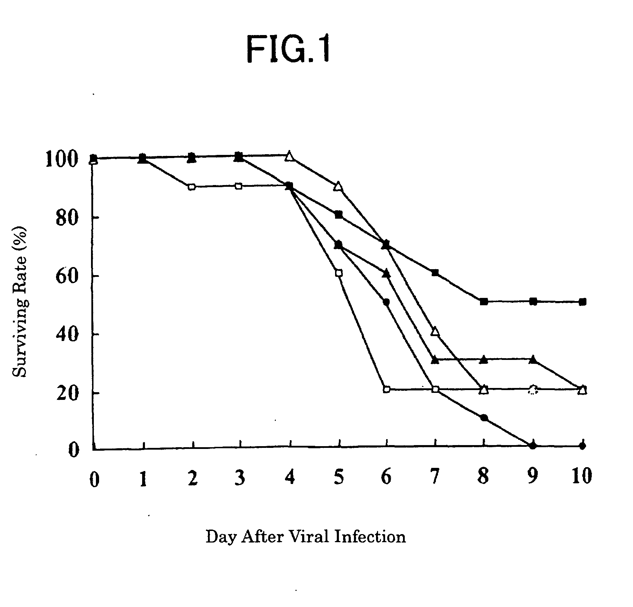

The results plotted on Fig. 1 clearly indicate that ambroxol increases the surviving rate of the mice infected with the influenza A/Aichi/68 (H3N2) virus. In respect of the results plotted on Fig. 1, mice were infected with 6.6 × 10⁴ PFU of influenza A/Aichi/68 (H3N2) virus and then the mice were injected with an aqueous common salt solution (●), 4 mg/mg/kg/day (▲), 10 mg/mg/kg/day (■), 20 mg/kg/day (Δ) and 30 mg/kg/day (□) of ambroxol, intraperitoneally. Thus the surviving rate of each group of test animals (each group consisted of 10 animals) was analyzed or monitored over 10 days.

As has been discussed above, there has been reported that ambroxol stimulates the lung and the main bronchus to secrete PS and that it has an anti-oxidative effect and anti-inflammatory characteristics. The effect of ambroxol on mice infected with the influenza A/Aichi/68 (H3N2) virus, which shows a high infectivity and a strong progressiveness, was analyzed or evaluated in the light of the foregoing knowledge. Mice each having a body weight ranging from 8 to 10 g were intra-nasally infected with influenza A viruses in an amount corresponding to the lethal dose thereof and ambroxol was intraperitoneally injected into these animals in a variety of doses twice a day. Ambroxol per se was not toxic up to a dose of 30 mg/kg/day.

A significant decrease of the body weight of each animal was observed after two days from the virus-infection and all of the test animals (n = 10), which had not been treated with ambroxol, were killed within 10 days. In the groups treated with ambroxol, the surviving rate of the infected mice was improved depending on the dose of ambroxol. More specifically, the surviving rate reached a peak value at a dose of 10 mg/kg/day, but the surviving rate-improving effect of ambroxol was reduced at a dose higher than that dose (see Fig. 1). When treating mice with ambroxol at a dose of 10 mg/kg/day, a half of the infected mice survived although they were infected with the lethal dose of the virus.

### 2) In the test animal groups treated with ambroxol, any virus-proliferation was inhibited.

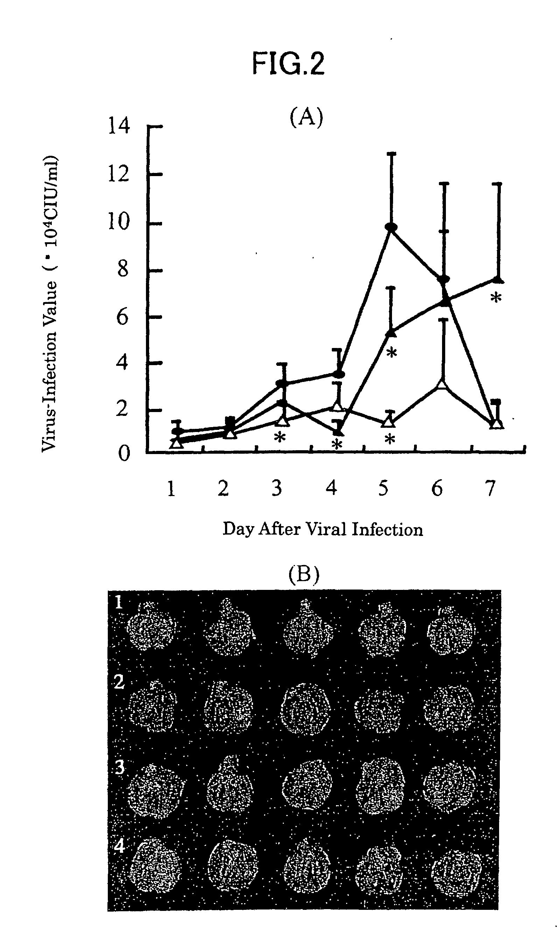

Fig. 2 shows the effect of ambroxol for inhibiting any virus-proliferation in BALF (A) and the lesions visually observed in the mice after 4 days from the influenza virus-infection (B).
A: Mice in each group (80 animals) were infected with influenza A/Aichi/68 (H3N2) virus and then treated with an aqueous common salt solution (•) and 10 mg/mg/kg/day (Δ) and 30 mg/kg/day (▲) of ambroxol as is described later in the explanation of Fig. 1. After the infection and the treatment, BALF was collected from 5 survived mice every day over 7 days. The titer of virus in BALF was determined according to the foregoing immunofluorescent cell-counting method (Ref. 20) and the results obtained were expressed in cell infecting unit (CIU). These data are expressed by the average±SD (n = 5). The significant difference between the values observed for mice treated with the aqueous common salt solution and ambroxol was determined by conducting Student's t-Test. *P<0.01.
B: Pulmonary lesions of mice visually observed for the lungs of the animals belonging to the un-infected group after 4 days (n = 5) (1), the lungs of the animals belonging to the group infected with influenza viruses and treated with the aqueous common salt solution (2), and the lungs of the animals belonging to the groups infected with influenza viruses and treated with 10 mg/kg/day (3) and 30 mg/kg/day of ambroxol.

The titer of virus in BALF was determined according to the foregoing immunofluorescent cell-counting method in order to elucidate the basic mechanism of ambroxol to improve the surviving rate of the infected mice.

After 2 days from the intranasal infection of mice with influenza A viruses, the viral titer in BALF began to increase, reached the maximum value after 5 days from the infection, the viral titer in BALF was rapidly reduced on and after the 6^{th} day and the titer observed on the 7^{th} day was almost identical to that observed after two days from the infection. It would be assumed that this is a result of the immunological reaction in the host (see Fig. 2A). In the group treated with ambroxol at a dose of 10 mg/kg/day, the virus-proliferation was significantly inhibited, but the virus-proliferation-inhibitory effect observed for the group treated with ambroxol at a dose of 30 mg/kg/day was inferior to that described above. The test animals were inspected for the pathological changes of the lungs or the visual pulmonary lesions on the 4^{th} day from the virus-infection (see Fig. 2B). There were observed, in the infected mice, severe and wide-spreading liver-like lesions on the lungs along with the rubefaction. On the other hand, in the group treated with ambroxol at a dose of 10 mg/kg/day, the pathological changes were distinctly reduced and the lesion-inhibitory effect of ambroxol at a dose of 30 mg/kg/day was lower than that specified above.

The virus-proliferation in the infected mice was almost ceased and the viruses were eliminated from the respiratory tract on the 7th day, but the pathological changes in the lung were maintained even after the virus-proliferation had been ceased with a slight progress and the animals were killed within 10 days. In order to elucidate the mechanism of the improvement in the surviving rate of the infected mice and that of the contribution of ambroxol to the virus-proliferation-inhibitory effect, the inventors of this invention investigated the effect of ambroxol on a variety of cellular factors and inflammatory cytokines present in BALF and controlling the replication of influenza viruses.

### 3) Effects of ambroxol on the concentrations of influenza virus-replication-promoting and -inhibitory factors present in the fluid secreted in the respiratory tract.

The trypsin-type protease secreted in the respiratory tract such as Tryptase Clara cleaves HA of influenza virus into HA1 and HA2 and as a result, the protease may activate the viral membrane-fusing ability and promote the viral replication (Ref. 6, 7). Endogeneous inhibitory substances such as MPI (Ref.8) and PS (Ref.9) inhibit this protease activity. For this reason, the inventors investigated the effect of ambroxol on the concentrations of these inhibitory substances in BALF. The results thus obtained are summarized in the following Table 1 (Effect of ambroxol on trypsin-like protease activity, PS and MPI in BALF originated from mice infected with influenza A viruses).

The trypsin-type protease is in general secreted in the un-infected mice and rats in an amount greater than those of the protease-inhibitory substances in the respiratory tract and therefore, the respiratory tract is always ready for the infection with influenza viruses (Refs. 6, 9). When these animals were infected with influenza viruses, the concentration of the trypsin-type protease reached the peak value after 6 days from the infection on the order of about 6.4 times that observed for the un-infected animals. In the group treated with ambroxol at a dose of 10 mg/kg/day, the secretion of the protease was already accelerated on the 1^{st} day and the level of the protease reached the peak value on the 5^{th} day from the initiation of the treatment. In the group to which ambroxol was administered at a dose of 30 mg/kg/day, the level of the protease was further increased, but it reached the peak level within a shorter period of time or on the 4^{th} day and thereafter, there was observed such a tendency that it was rapidly reduced. Even in the un-infected mice, it was recognized that the secretion of the trypsin-type protease in BALF was promoted due to the action of ambroxol (see the data listed in Table 2). In the test animal groups to which ambroxol was administered at doses of 10 and 30 mg/kg/day, the trypsin-type protease levels reached their peak values on the 4^{th} day or up to 2.2 times and 2.4 times that observed for the un-infected animals.

The inventors further investigated the effect of ambroxol on the levels of SP-A and MPI [see the data listed in the following Tables 1 and 2 (the effect of ambroxol on the trypsin-like protease activity, PS and MTI in the mouse BALF in the influenza virus-infected group and the un-infected group)].

The infection with influenza viruses increased the levels of bio-protective substances or SP-A and MPI each having an anti-influenzal activity. More specifically, the levels thereof reached their peak values on the 6^{th} day, which were on the order of 6 times and 4.4 times that observed for the un-infected animals. The treatment of the infected animal group with ambroxol permitted rapid and significant increase in the concentrations of MPI and SP-A after one day from the administration of ambroxol and reached their peak values on the order of 9 to 10 times and 8.4 times those observed for the un-infected animals. In the group in which infected mice were treated with ambroxol at a dose of 10 mg/kg/day, the levels of MPI and SP-A were rapidly increased on the 1^{st} day, thereafter they were gradually increased, they reached the peak values on the 5^{th} day and they were maintained at high levels until the 7^{th} day. However, when mice were treated with 30 mg/kg/day of ambroxol, the levels of MPI and SP-A were rapidly increased on the 1^{st} day, reached the maximum values on the 4^{th} day and then MPI and SP-A rapidly disappeared. It was also observed that even the secretion of SP-A and MPI in the un-infected group of mice were lightly promoted by the administration of ambroxol.

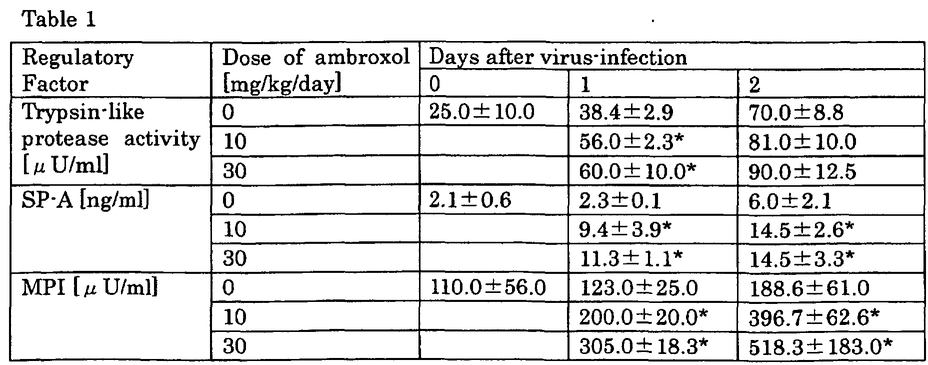

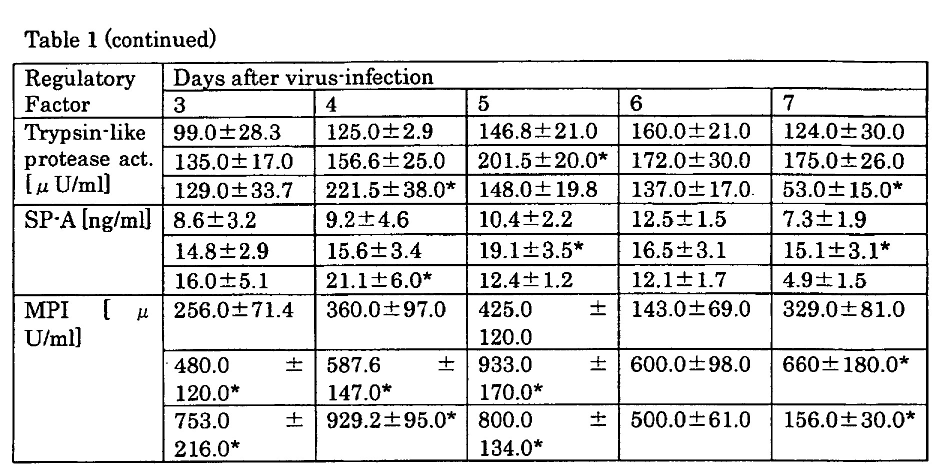

The data in this table are expressed in terms of the average±SD. n = 5 for each data. The significant difference between the ambroxol-treated group and the group free of such a treatment, for each day, was evaluated by the Student's t-Test. *P<0.05.

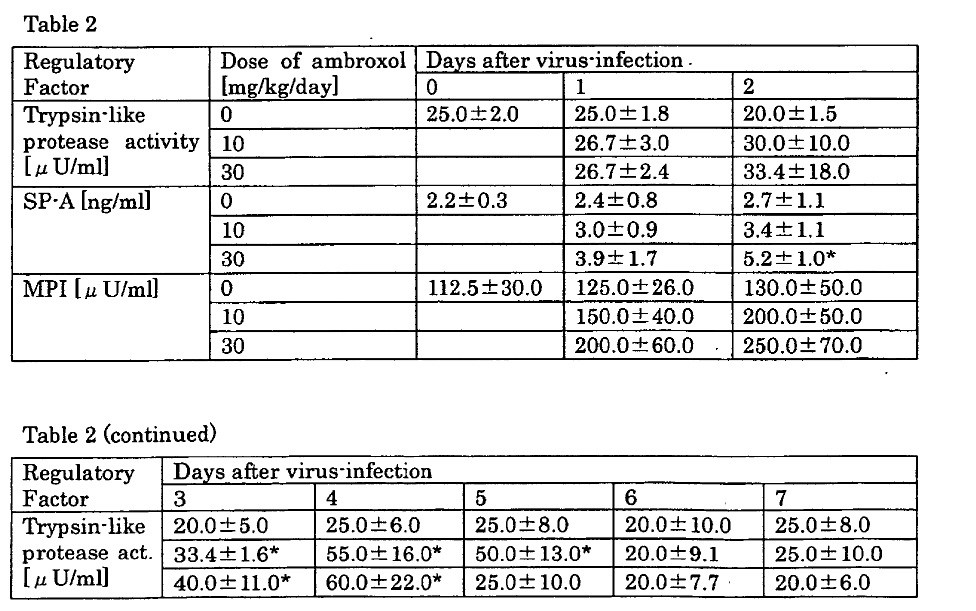

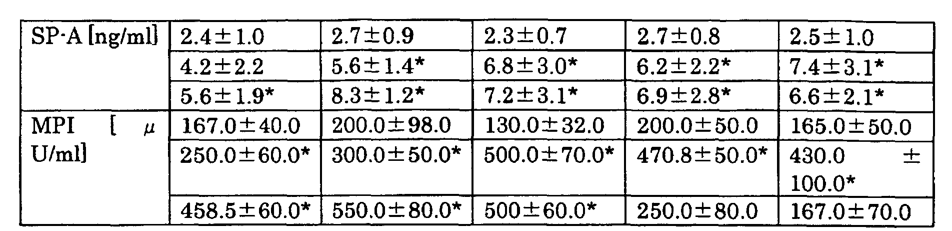

The data in this table are expressed in terms of the average±SD. n = 5 for each data. The significant difference between the ambroxol-treated group and the group free of such a treatment, for each day, was evaluated by the Student's t-Test. *P<0.05.

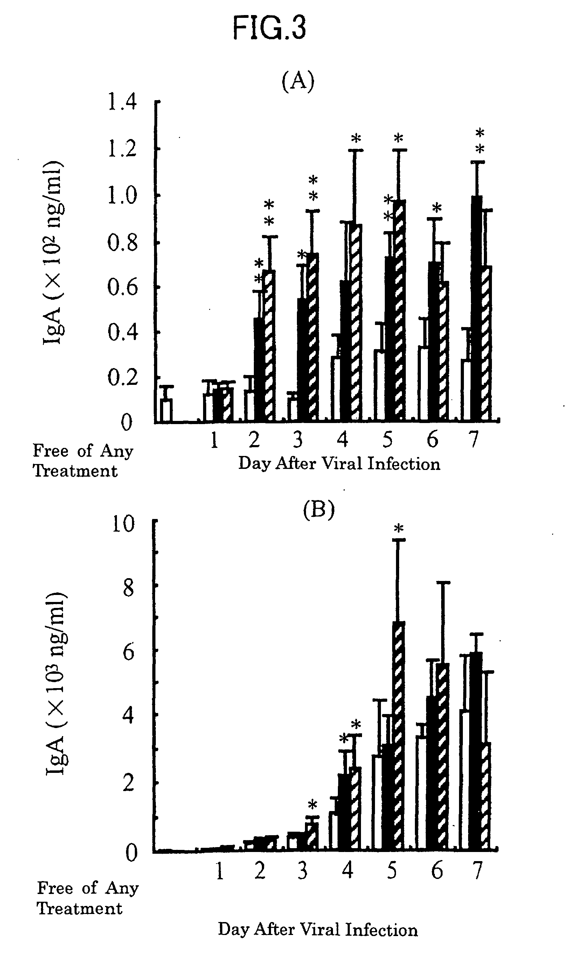

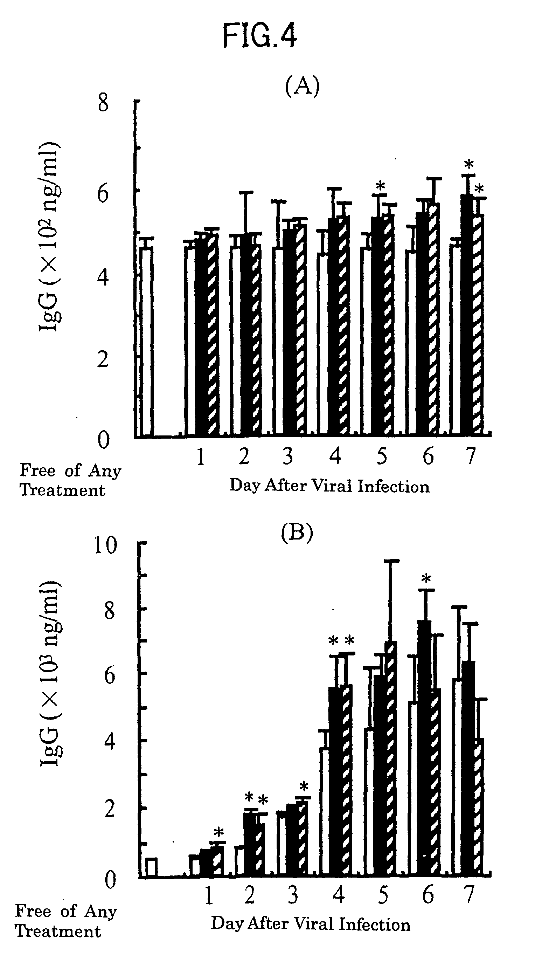

Then the effect of ambroxol on the secretion of mucosal immunoglobulins IgA and IgG in BALF was investigated (see Figs. 3 and 4).

Fig. 3 shows the secretion-promoting effect of ambroxol on the level of mucosal immunoglobulin IgA in BALF of (A) a group of un-infected mice and (B) a group of mice infected with influenza A virus. In respect of the data shown in this figure, the levels of IgA present in BALF of mice belonging to the un-infected group (A) and the infected group (B) treated with an aqueous common salt solution (white bar), and 10 mg/kg/day (black bar) and 30 mg/kg/day (shadowed bar) of ambroxol were monitored over 7 days. These data are expressed in terms of the average±SD (n = 5). According to the Student's t-Test, the significant differences between the mice treated with the aqueous common salt solution and ambroxol were found to be *P<0.05 and **P<0.01.

Fig. 4 shows the secretion-promoting effect of ambroxol on the level of mucosal immunoglobulin IgG in BALF of (A) a group of un-infected mice and (B) a group of mice infected with influenza A virus. In respect of the data shown in this figure, the levels of IgA present in BALF of mice belonging to the un-infected group (A) and the infected group (B) treated with an aqueous common salt solution (white bar), and 10 mg/kg/day (black bar) and 30 mg/kg/day (shadowed bar) of ambroxol were monitored over 7 days. These data are expressed in terms of the average ±SD (n = 5). According to the Student's t-Test, the significant difference between the mice treated with the aqueous common salt solution and ambroxol was found to be *P<0.05.

The intra-nasal inoculation of influenza virus could considerably promote the secretion of mucous immunoglobulins IgA and IgG. The levels of these antibodies has been recognized to be correlated with the degree of the virus-proliferation-inhibition (Liew FY, Russell SM, Appleyard G, Brand CM, Beale J, Cross-protection in mice infected with influenza A virus by the respiratory route is correlated with local IgA rather than serum antibody or cytotoxic T cell reactivity, Eur. J. Immunol., 1984, 14: 350-356; Tamura S, Funato H, Hirabayash Y, et al., Functional role of respiratory tract haemagglutinin-specific IgA antibodies in protection against influenza, Vaccine, 1990, 8: 479-485).

The IgA concentration in BALF derived from the group of un-infected mice is very low on the order of 10.3 ± 6.6 ng/ml, while the IgG concentration is relatively high on the order of 460 ± 26.2 nm/ml. This relatively high IgG level may be due to the diffusion thereof from the serum to the fluid secreted in the respiratory tract (see Figs. 3A and 4A). The treatment of the group of un-infected mice with 10 and 30 mg/kg/day of ambroxol promoted the IgA secretion. More specifically, the treatment increased the concentration thereof to a level of about 10 times that observed for the group free of such a treatment on the 7^{th} and 5^{th} day, respectively, from the initiation of the treatment and the treatment slightly increased the concentration of IgG to a level of about 1.2 time that observed for the group free of such a treatment on the 7t^{h} and 6^{th} day from the initiation of the treatment. If infecting mice with influenza virus, the concentrations of IgA and IgG in BALF substantially increased after one to two days from the infection, the levels thereof reached their peaks or the level of IgA on the 7^{th} day was found to be about 400 times and the level of IgG on the 6^{th} day was found to be about 11 times those observed for the group free of such a treatment (Figs. 3B and 4B). The treatment of the infected mice with 10 and 30 mg/kg/day of ambroxol significantly increased the IgA levels on the 7^{th} day and 5^{th} day to about 600 times and 700 times, respectively, that observed for the basic concentration of IgA. On the other hand, the treatment of the infected mice with 10 and 30 mg/kg/day of ambroxol medially stimulated the IgG secretion in the infected mice or the concentrations thereof on the 6^{th} day and 5^{th} day were found to be about 16 times and 15 times that of the basic concentration of IgG. These results might be obtained because of the considerable promotion of the mucosal immunoglobulin IgA secretion and the medium promotion of the IgG secretion, which were induced by the infection, through the treatment with ambroxol. Therefore, it would be recognized that these increases of the immunoglobulin levels were the results of the virus-proliferation-inhibitory effect of ambroxol in the respiratory tract.

### 5) Effect of Ambroxol on Release of Cytokines

There has been reported that ambroxol inhibits, in vitro, any release of inflammatory cytokines such as TNF-α, IL-2, IL-1, IL-4, IL-13 and IFN- γ (Pfeifer S, Zissel G, Kienast K, Muller-Quernheim J., Reduction of cytokine release from blood and bronchoalveolar mononuclear cells by ambroxol, Eur. J. Med. Res., 1997, 2: 129-132; Gibbs BF, Schmutzler W, Vollrath IB, Brostharardt P, Braam U, Wolff HH, Zadlo-Klarwasser G., Ambroxol inhibits the release of histamine, leukotrienes and cytokines from human leukocytes and mast cells, Inflamm. Res. 1999; 48: 86-93). To determine the anti-inflammatory effect of ambroxol in the mice infected with influenza virus, the inventors of this invention investigated or analyzed the levels of mucosal immune-promoting cytokines, for instance, inflammatory cytokines such as TNF- α, IL-4 and INF- γ , IL-6 and IL-12 (Boyaka PN, Marinaro M, Jackson R, Menon S, Kiyono H, Jirillo E, McGhee JR., IL-12 is an effective adjuvant for induction of mucosal immunity, J. Immunol., 1999,162:122-128). The results thus obtained are listed in the following Table 3 (the effect of ambroxol on the cytokines such as TNF- α, IL-12, INF- γ and IL-6 in BALF derived from the mice infected with influenza A/Aichi/68 (H3N2) viruses.

The levels of the cytokines present in BALF derived from the un-infected mice were lower than the detection limits. The infection with influenza virus significantly induced the secretion of all of the cytokines examined except for IL-4 in BALF, although the patterns thereof as a function of the elapsed time were different from one another. More specifically, when the animals were infected with influenza virus, the level of TNF- α was initially increased or it reached the peak level on the 1^{st} day, thereafter it was rapidly reduced and it reached a secondary small peak on the 6^{th} day. The level of IL-6 was also rapidly increased on the 1^{st} day after the infection, the level was maintained at such a high level and reached its peak value on the 5^{th} day, but began to undergo rapid reduction on the 7^{th} day after the infection. The concentrations of IL-12 and INF- γ were gradually increased after the administration of ambroxol and reached the respective peak values on the 4^{th} and 6^{th} days after the administration thereof. However, IL-4 was not detected in BALF derived from the infected mice over seven days examined (data were not shown). When the infected mice were treated with ambroxol, there were observed the TNF- α, INF- γ and IL-12-secretion-inhibitory effects on 3^{rd} to 5^{th} day, the 1^{st} day and 4^{th} day, respectively, after the initiation of the treatment with ambroxol, but the inhibitory effect was not always observed during the treatment with ambroxol. On the other hand, the level of IL-6 in BALF derived from the infected mice was increased on the 4^{th} and 6^{th} days after the initiation of the treatment with ambroxol.

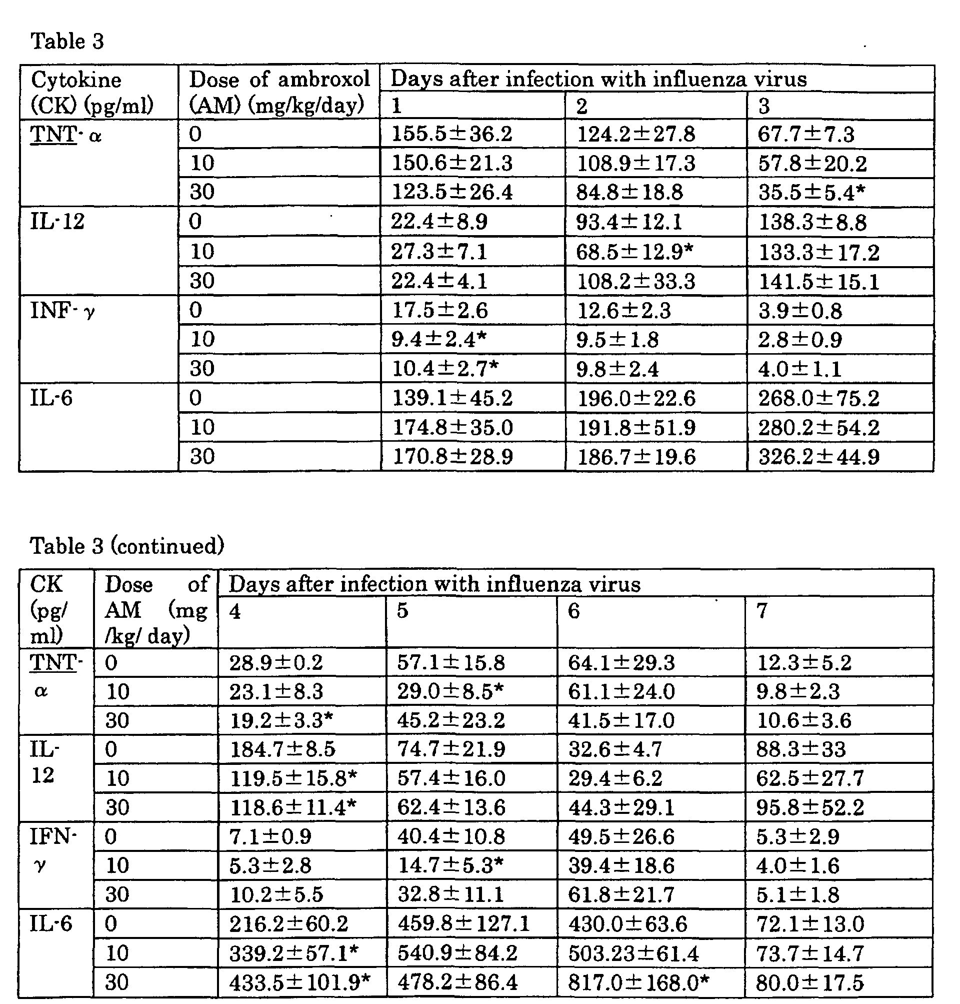

The data in this table are expressed in terms of the average±SD. n = 5 for each data. The significant difference between the ambroxol-treated group and the group free of such a treatment, for each day, was evaluated by the Student's t-Test. *P<0.05.

### <Consideration>

In this study, it has been concluded that ambroxol significantly inhibits the influenza virus-proliferation in the respiratory tract and likewise improves the surviving rate of mice infected with the lethal dose of influenza A/Aichi/68 (H3N2) virus. Influenza virus shows organ specificity to the respiratory tract and the pathogenicity and replication thereof are determined by a variety of factors derived from the host cell and the immune responses of T-cells and B-cells.

There have been reported that, in the respiratory tracts of animals, the trypsin-type proteases such as tryptase Clara serves as a cellular factor, which promotes the influenza virus-replication (Kido H, Yokogoshi Y, Sakai K, Tashiro M, Kishino Y, Fukutomi A, Kutunuma N., Isolation and characterization of a novel trypsin-like protease found in rat bronchiolar epithelial Clara cells, J. Biol. Chem., 1992; 267:13573-13579; Tashiro M, Yokogoshi Y, Tobita K, Seto JT, Rott R, Kido H. Tryptase Clara, an activating protease for Sendai virus in rat lungs, is involved in pneumopathogenicity, J. Virol., 1992, 66: 7211-7216) and that on the other hand, factors capable of inhibiting the viral proliferation are MPI as an inhibitor of proteases (Beppu Y, Imamura Y, Tashiro M, Towatari T, Ariga H, Kido H., Human Mucus protease inhibitor in airway fluids is a potential defensive compound against infection with influenza A and Sendai viruses, J. Biochem., 1997, 121: 309-316) and PS, which adsorbs proteases to thus inhibit the activity thereof (Kido H, Sakai K, Kishino Y, Tashiro M., A pulmonary surfactant is a potential endogenous inhibitor of proteolytic activation of Sendai virus and influenza virus, FEBS Lett., 1993, 322: 115-119). The concentration of the trypsin-type protease in the usual environment of the respiratory tract is maintained at the level permitting the infection with and proliferation of influenza virus and is higher than those of inhibitors present therein.

PS covering the pulmonary alveolar epithelium is linked with tryptase Clara to thus inhibit the activity of the protease (Kido H, Sakai K, Kishino Y, Tashiro M., A pulmonary surfactant is a potential endogenous inhibitor of proteolytic activation of Sendai virus and influenza virus, FEBS Lett., 1993, 322: 115-119; Kido H, Murakami M, Oba K, Chen Y, Towatari T., Cellular proteinases trigger the infectivity of the influenza A and Sendai viruses, Mol. Cells, 1999, 9: 235-244). The infection with influenza virus promoted the secretion of both trypsin-type protease required for the proliferation of the virus and the inhibitor thereof. Moreover, when the infected mice were treated with ambroxol, the secretion of the foregoing substances was further promoted significantly, but the effect changed the balance between the protease and the inhibitor. The concentration of the protease induced by the viral infection was further increased to a level of 1.3 to 1.4 times, while those of SP-A and MPI were increased to a level of 1.5 to 1.7 times and 1.9 times, respectively by the treatment with ambroxol. These results clearly indicate that the treatment with ambroxol permits the rate of the substances showing virus-proliferation-inhibitory effects and present in the fluid secreted in the respiratory tract of the infected mice and that the treatment improves the environment in the respiratory tract in such a manner that it has a high virus-proliferation-inhibitory tendency as compared with the environment in the respiratory tract of un-treated infected mice.

Moreover, ambroxol had an effect of promoting or increasing the release of mucosal immunoglobulins IgA and IgG in the infected and un-infected mice as shown in Fig. 3. This drug also medially promoted the secretion of IgG (Fig. 4). Ambroxol likewise promoted the release of IgA even in the un-infected mice. More specifically, the concentration of IgA was increased to about 10 times the basic concentration thereof and that of IgG was increased to 1.2 times the basic concentration. After the viral infection, the concentrations of IgA and IgG in BALF were considerably increased, but the treatment of the infected mice with ambroxol could increase the concentrations of IgA and IgG to levels of about 1.5-1.8 times and 1.45 times, respectively, the maximum concentrations thereof induced through the viral infection. This clearly indicates that the increases in the concentrations of IgA and IgG due to the treatment with ambroxol play an important role in the improvement of the surviving rate of the infected mice.

The precise mechanism of ambroxol to promote the secretion of a variety of factors such as IgA and IgG, SP-A, MPI and trypsin-type proteases has not yet been clearly elucidated, but the foregoing facts suggest that ambroxol would stimulate a plurality of target cells of the upper and lower respiratory tracts. When mice were treated with ambroxol in the optimum dose of 10 mg/kg/day, the concentrations of the virus-proliferation-inhibitory substances and those of immunoglobulins in the respiratory tract were gradually increased with the elapsed time and the concentrations thereof were maintained at such high levels on the 7th day when virus-replication was ceased. However, the treatment with a higher dose of ambroxol rapidly increased the concentrations of these substances and the latter quickly reached their peak levels on the 4^{th} to 5^{th} day, but such high levels thereof could not be maintained throughout the viral infection. This would be a cause of such a low virus-proliferation-inhibitory effect of ambroxol at a dose of 30 mg/kg/day. These results indicate that ambroxol promotes the secretion of the inhibitory substances and the trypsin-type proteases rather than promotes the synthesis of these substances in the fluid of the respiratory tract. Moreover, one would be recognized that these virus-proliferation-inhibitory substances should be maintained at such high levels over 7 days to improve the surviving rate of the infected mice.

The viral proliferation was ceased on the 7^{th} day (see Fig. 2), but the inflammation of the lung was maintained and even made slow progress. Recently, a variety of studies have made it clear that ambroxol possesses an anti-inflammatory effect (Gillissen A, Scharling B, Jaworska M, Bertling A, Rasche K, Schultze- Werninghaus G., Oxidant scavenger function of ambroxol in vitro: a comparison with N-acetylcysteine AC, Res. Exp. Med. (Berl.), 1997, 196: 389-398) and permits the reduction of the ability of producing inflammatory cytokines (Pfeifer S, Zissel G, Kienast K, Muller-Quernheim J., Reduction of cytokine release from blood and bronchoalveolar mononuclear cells by ambroxol, Eur. J. Med. Res., 1997, 2: 129-132; Gibbs BF, Schmutzler W, Vollrath IB, Brostharardt P, Braam U, Wolff HH, Zadlo-Klarwasser G., Ambroxol inhibits the release of histamine, leukotrienes and cytokines from human leukocytes and mast cells, Inflamm. Res., 1999, 48: 86-93). In this study, it has been recognized that ambroxol inhibits the levels of inflammatory cytokines or THF- α and IFN- γ in the fluid secreted in the respiratory tract of the infected mice, but the effect is not always observed throughout the viral infection. Moreover, it has been reported that both IL-6 and IL-12 possess an effect of promoting the mucous immune and in particular, it has been reported that IL-12 has an effect of promoting the production of the mucous immunoglobulin IgA (Boyaka PN, Marinaro M, Jackson R, Menon S, Kiyono H, Jirillo E, McGhee JR., IL-12 is an effective adjuvant for induction of mucosal immunity, J. Immunol., 1999,162:122-128). The treatment of the infected mice with ambroxol increased the levels of IL-6 in BALF on the 4^{th} day and 6^{th} day and temporarily inhibited the level of IL-12 on the 4^{th} day.

On the one hand, ambroxol showed effects, which are unfavorable for the bio- defensive system against the influenza virus, such as an effect of increasing the level of the trypsin-type proteases and an effect of temporal inhibition of any release of IL-12, but the treatment with ambroxol, on the whole, considerably increased the concentrations of biological factors showing the virus-proliferation-inhibitory effect in the fluid secreted in the respiratory tract and as a result, it could inhibit the viral proliferation in the respiratory tract to thus significantly improve the surviving rate of the mice infected with influenza viruses. Among these, the effect of ambroxol involved in the inhibition of influenza virus proliferation would be proved by the increase in the concentrations of, for instance, SP-A, MPI, IgA and IgG in the respiratory tract and the inhibition of any release of inflammatory cytokines in the respiratory tract. These results suggest that ambroxol can clinically be used for the treatment of subjects infected with influenza A virus or prevention of influenza A virus infection.

In general, there is such a tendency that influenza prevails and the prevalence thereof, in most cases, goes into headlines. Therefore, the anti-influenzal agent of the present invention can be used as a therapeutic agent immediately after the prevalence was informed, and also even after the infection. In particular, the agent for treating subjects infected with influenza virus or preventing the infection therewith according to the present invention can effectively be applied to the treatment or prevention of diseases caused by causal viruses, which have outer membrane glycoproteins and infect the respiratory tract to thus undergo proliferation, such as influenza viruses.

## Claims

1. An anti-influenzal agent comprising ambroxol, bromhexin or a pharmaceutically acceptable salt thereof as an effective component.

2. An anti-influenzal agent comprisng ambroxol or a pharmaceutically acceptable salt thereof as an effective component.

3. The anti-influenzal agent of claims 1 or 2, wherein it shows its anti-influenzal function through promoting the secretion of a bio-factor showing anti-viral function against influenza viruses and contained in a liquid secreted from the respiratory tract.

4. The anti-influenzal agent of claim 3, wherein it inhibits any proliferation of influenza viruses in the respiratory tract, through promoting the secretion of an inhibitor against the protease in the respiratory tract, which induces influenza virus infection.

5. The anti-influenzal agent of claim 4, wherein it further inhibits the proliferation of influenza viruses in the respiratory tract through promoting the secretion of IgA and IgG as mucosal immune substances.

6. The anti-influenzal agent as set forth in any one of claims 3 to 5, wherein it further inhibits any secretion of release of inflammatory cytokine in the respiratory tract.

7. The anti-influenzal agent as set forth in any one of claims 1 to 6, wherein it is an agent for treating or preventing influenza virus-infectious diseases.

8. Use of ambroxol, bromhexin or a pharmaceutically acceptable salt thereof for the preparation of anti-influenzal agent.

9. A method of treating influenza virus-infectious diseases which comprises administering an anti-influenzal agent comprising ambroxol, bromhexin or a pharmaceutically acceptable salt thereof as an effective component to patients suffering from the diseases.
